# FASCICULE DE BREVET EUROPEEN

(11) **EP 0 516 510 B1**
(45) Date de publication et mention de la délivrance du brevet: **06.09.1995**
(21) Numéro de dépôt: 92401380.8
(22) Date de dépôt: 21.05.1992
(51) Int. Cl.: A61M 15/00

(54) **Inhalateur de poudres**
Pulverinhalator
Powder inhaler

(30) Priorité: 30.05.1991 FR 9106531
(43) Date de publication de la demande: 02.12.1992
(73) Titulaire: S O F A B, 76450 Le Tréport (FR)
(72) Inventeur: Bougamont, Jean-Louis, F-76260 Eu (FR); Behar, Alain, F-76260 Eu (FR)
(74) Mandataire: Eudes, Marcel

(56) Documents cités:
- WO-A-90/07351
- WO-A-90/13327
- WO-A-90/13328
- US-A- 2 587 215

## Description

Un certain nombre de médicaments formulés en poudre sont destinés à être inhalés, ceci par voie buccale. Il s'agit en particulier de médicaments de l'asthme ou d'autres affections pulmonaires. Le document US-A-2 587 215 décrit un inhalateur pour médicaments en poudre à fonctionnement manuel.

Une difficulté de ce genre d'appareils est alors que le patient parvient souvent mal à synchroniser son aspiration sur le fonctionnement manuel du distributeur, c'est-à-dire sur le moment où la poudre se trouve expulsée et parfois dispersée dans un tourbillon créé par celui-ci.

L'invention a pour objet de fournir un inhalateur apte à garantir à la fois un dosage correct de la poudre donc du principe actif et une bonne utilisation par une aspiration aussi précise que possible en direction des voies respiratoires ; elle propose à cette fin un appareil commandé par cette aspiration elle-même.

Plus précisément, cet appareil se compose essentiellement d'un corps doté d'une embouchure d'aspiration buccale, associé à un organe à déplacement alternatif jouant le rôle d'un tiroir distributeur mû par un piston mobile sous deux actions opposées, l'une répulsive d'un moyen élastique, l'autre attractive, cet organe porte le long de sa paroi latérale au moins une cavité de transfert que son mouvement mettra en relation directe, en fin de répulsion avec le débouché inférieur d'une réserve de produit montée sur le corps, caractérisé en ce que en fin d'attraction la cavité de transfert de la poudre est mise en relation directe avec le débouché inférieur d'une prise d'air externe que la cavité met ainsi en relation par un canal adéquat avec l'embouchure le mouvement d'attraction étant du à la dépression causée par une inspiration du patient à travers l'embouchure.

Selon un perfectionnement avantageux, le piston ferme également une chambre que son recul met sous vide et la cavité met aussi, en fin de course répulsive, la réserve en relation avec cette chambre de mise sous vide, de façon à y créer une brève succion qui améliorera le remplissage si la poudre possède un écoulement imparfait.

De plus un passage convenablement étranglé peut mettre ladite chambre en communication avec l'extérieur de façon à équilibrer en partie ses variations de pression interne, ceci se faisant notamment à travers l'embouchure.

De préférence les communications respectives de la cavité s'ouvrent un peu avant la fin de chacune des phases successives et, de façon avantageuse, la succion au passage des orifices est autorisée dans un seul sens par un clapet, tiroir ou manchon que commande avec jeu le mouvement du piston ; en soi, les modes de construction possibles d'un tel mécanisme sont bien connus à travers divers types de pompes miniaturisées.

Enfin, au mieux et pour faciliter l'exécution des passages, le corps revêt la forme d'un boîtier injecté enfermant le piston, ses organes annexes et, au besoin, la réserve de produit.

Une description détaillée sera donnée ci-dessous en référence à un exemple décrit à travers des dessins volontairement schématisés pour en faire apparaître clairement les circuits, permettant une compréhension plus complète du dispositif et de son fonctionnement. Le spécialiste des pompes et distributeurs trouvera de nombreuses variantes constructives à cette structure, dans la disposition des organes et notamment l'exécution du clapet ou celle des divers canaux, obtenus par association de perçages ou de saignées pratiqués sur les différentes pièces de façon à éviter en particulier les difficultés de moulage.

Ces dessins montrent schématiquement :
- figure 1 : une version élémentaire du dispositif,
- figure 2 : une vue en bout de cette version,
- figure 3 : une coupe par III-III de la figure 1,
- figure 4 : une variante avec succion de la dose de produit,
- figure 5 : une coupe par V-V de la figure 4.

Comme indiqué plus haut, l'appareil décrit visera à permettre une inhalation efficace d'un médicament en poudre en subordonnant sa distribution à un mouvement d'aspiration du patient car son fonctionnement sera directement commandé par les mouvements respiratoires de ce dernier.

Dans le corps 1 de l'appareil est placé un piston 2 de section appréciable, sur une paroi latérale duquel est amenagé un logement simple ou multiple formant une cavité 3 dont le volume correspond à la dose de médicament désirée.

Le corps 1 est porteur d'une embouchure qui reçoit un embout buccal 4, tandis qu'une bague ou un diaphragme 5, ouvert à l'air libre, enferme le piston 2, qu'un ressort 6 renvoie en arrière contre cette butée en position de repos. La cavité 3 est alors placée en face d'un puits 7 dans lequel se trouve enfermée la réserve 8 de médicament. La présentation de l'appareil conduisant en fait à placer sur le dessus la réserve 8, la poudre tombe par gravité dans le logement et remplit la cavité.

Le piston coulisse dans une chambre 9, sa lèvre 2a faisant étanchéité ; le schéma le montre en fin de la phase d'aspiration. Il est plein, la cavité 3 est disposée sur sa tige 2b et le ressort 6, placé dans une chambre 10, agit sur cette tige ; mais il est clair que l'on pourrait adopter des dispositions différentes et notamment monter axialement la réserve ou placer le ressort dans la chambre 9 et non dans la chambre 10. De même les sections des divers organes sont représentées uniformément circulaires et sans nullement préjuger des proportions, alors qu'elles seront en réalité adaptées à l'existence d'un socle et d'empreintes de doigts facilitant et orientant la prise de l'appareil, à la forme de la bouche, à l'écoulement de la poudre ...

Un canal 9a fait communiquer la chambre principale 9 du piston avec l'embouchure ; si le patient, qui a préalablement oté le capot de protection 11, place l'embout buccal 4 dans sa bouche et inspire, le canal 9a transmet à la chambre 9 la dépression que provoque cette inspiration : le piston 2 avance en comprimant le ressort 6.

De même un orifice 10a peut mettre la chambre secondaire 10 à l'air libre ou, mieux, comme le montre le dessin, être relié à l'espace interne de l'embouchure de façon que l'effort inhalatoire du patient soit transmis à toute la section transversale du piston.

L'un au moins des orifices peut en outre être calibré de façon à amortir les déplacements de l'organe mobile, en particulier l'orifice 10a de la chambre secondaire, celle-ci jouant alors un rôle de frein autant que de moteur.

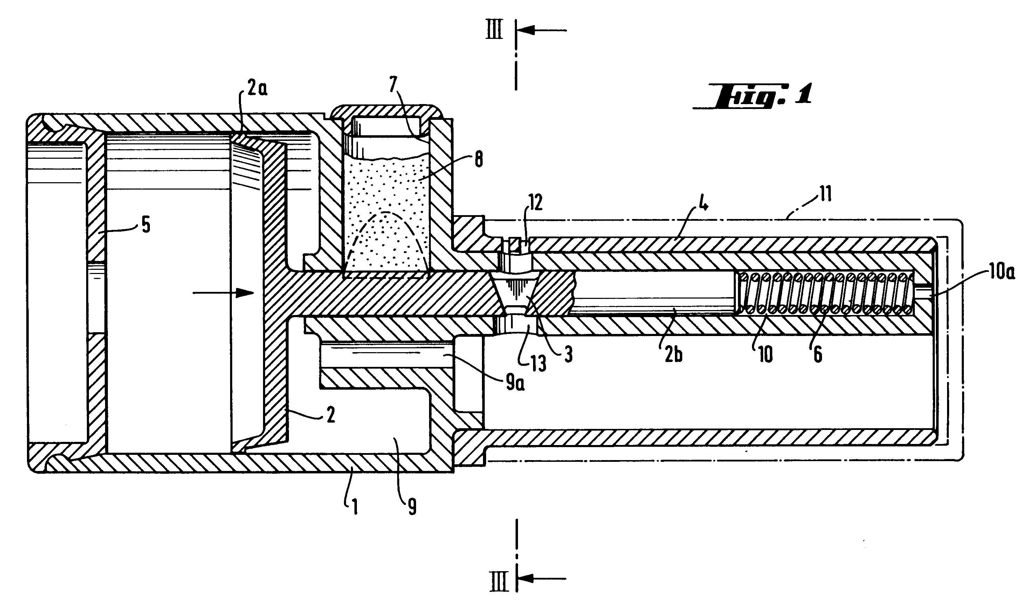

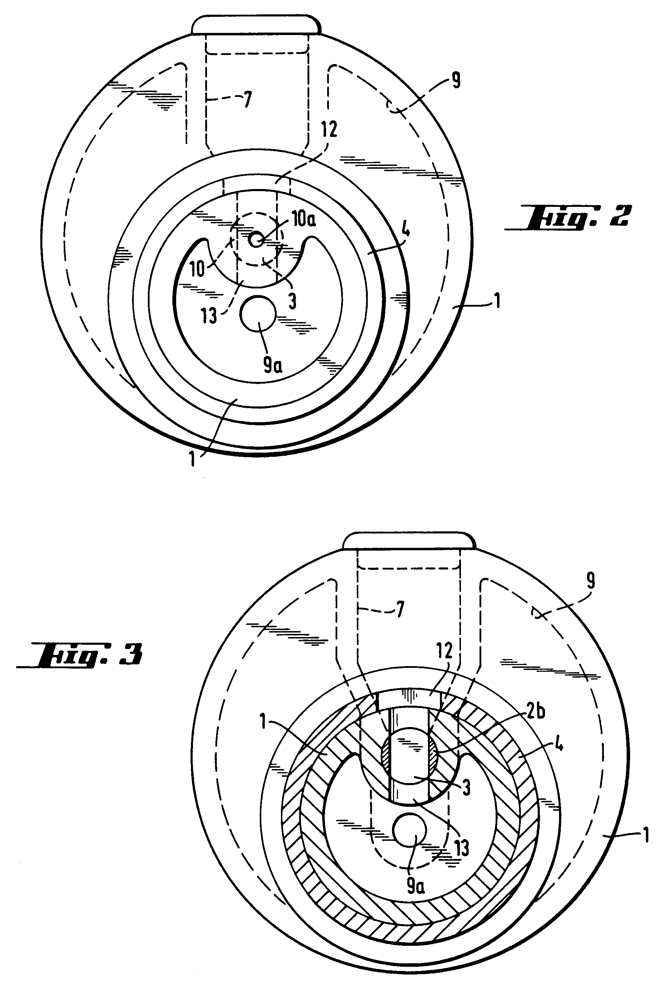

Dans la dernière partie de la course d'avance du piston, la cavité 3 vient, comme le montrent les figures 1 et 3, déboucher à la fois sur une prise d'air 12 et sur un passage opposé 13 qui la fait communiquer avec l'intérieur de l'embout, dont l'espace intérieur se trouve mis à l'air libre avec une perte de charge suffisante et, l'inspiration du patient continuant, le courant d'air ainsi créé chasse la poudre vers l'embout où elle se trouve brassée, puis vers la bouche du patient d'où elle est inhalée. En jouant sur la forme des passages successifs, il est possible d'obtenir un entraînement plus ou moins durable au fur et à mesure du passage de la cavité devant la prise.

Dès que l'aspiration cesse, le ressort 6 renvoie le piston en position de repos, ce qui permet à une nouvelle dose de poudre de pénétrer dans la cavité 3, aidée par le léger choc en butée au retour. Il convient donc que ce ressort possède une force suffisante pour être à même de verrouiller l'orifice mais qu'il ne soit pas trop puissant pour les forces du patient, donc que le piston ait au total une section efficace suffisante.

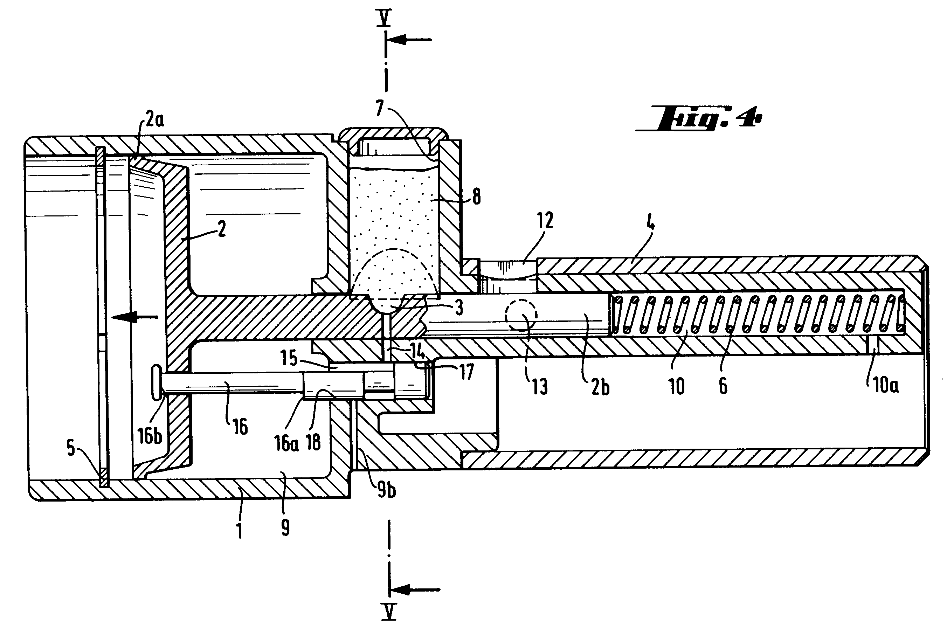

Le schéma de la figure 4 introduit une variante destinée à améliorer au besoin le remplissage initial de la cavité par la poudre. Elle consiste essentiellement en l'adjonction d'un étroit canal apte, comme le montre la figure, à déboucher sur la cavité 3 lorsque celle-ci arrive au voisinage de sa position de repos pour la relier à l'une des chambres du piston 2 : on comprend que le déplacement du piston vers la gauche de la figure lors de son recul vers cette position peut alors créer sur la poudre une brève succion qui aide celle-ci à descendre.

Sur le dessin, il s'agit pour la commodité du schéma d'un canal 14 atteignant la chambre 9 par un puits 15 : en fait il convient de relier la cavité 3 à une chambre secondaire, c'est-à-dire soit de tracer le canal 14 entre elle-même et le puits 7 d'une part, la chambre 10 d'autre part, soit, gardant le dessin actuel, d'en modifier les proportions pour inverser les rôles relatifs des deux chambres. C'est que l'effet de succion dépend essentiellement de la valeur de la dépression que la force du ressort peut engendrer dans la chambre correspondante, valeur d'autant plus élevée que la section de cette dernière représente une fraction plus faible de la section totale efficace du piston.

Il faut aussi que le canal 9a ou 10a correspondant soit assez étroit : à la limite on peut envisager de le fermer entièrement, le rééquilibrage des pressions au repos intervenant alors plus lentement, par le jeu des imperfections d'étanchéité : pour éviter toutefois de créer dans la phase d'avance, lors de la prise du médicament, une contre-pression nuisible, il devient alors particulièrement utile de donner au joint 2a du piston la forme de la lèvre tournée vers l'arrière que montre le dessin, ce qui lui permet de se substituer en somme audit canal lors du fonctionnement, jouant en outre un rôle de clapet de refoulement.

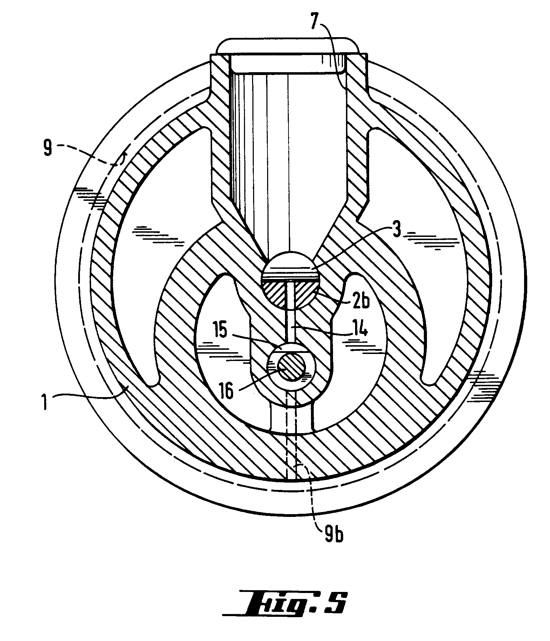

Les figures 4 et 5 montrent l'adjonction d'une sécurité complémentaire, sous la forme d'un clapet à tiroir, schématisé en 16 mais qui pourrait aussi bien revêtir la forme classique d'un manchon ou d'un insert situé le long de la tige 2b. Entraîné par le piston avec jeu entre deux butées 16a et 16b, sa portée 17 condamne le canal de succion 14 lors de l'essentiel de la course aller correspondant à la phase d'aspiration pour interdire tout refoulement au franchissement de son débouché par la cavité 3 ; elle n'en démasque le passage qu'à la fin pour le laisser jouer son rôle lors de la course de retour comme le montre la figure 4, puis se refermer au repos.

Ce clapet peut en outre être doté d'une portée 18, dessinée de façon à lui faire jouer un rôle de distributeur mettant par un canal 9b la chambre de succion à l'air libre lors du mouvement aller.

## Revendications

1. Inhalateur de produits en poudre, se composant essentiellement d'un corps (1) doté d'une embouchure d'aspiration buccale, associé à un organe à déplacement alternatif (2) jouant le rôle d'un tiroir mû par un piston, mobile sous deux actions opposées, l'une répulsive d'un moyen élastique (6), l'autre attractive, cet organe portant le long de sa paroi latérale au moins une cavité de transfert (3) que son mouvement mettra en relation directe, en fin de répulsion avec le débouché inférieur d'une réserve de produit (8) montée sur le corps, caractérisé en ce que en fin d'attraction la cavité de transfert de la poudre est mise en relation directe avec le débouché inférieur d'une prise d'air externe (12) que la cavité met ainsi en relation par un canal adéquat (13) avec l'embouchure, le mouvement d'attraction étant du a la dépression causée par l'inspiration du patient à travers l'embouchure.

2. Inhalateur selon la revendication 1, caractérisé en ce que le piston ferme également une chambre (9-10) que sa répulsion met sous vide, et qu'en fin de course répulsive, la cavité met aussi la réserve en relation avec cette chambre de mise sous vide.

3. Inhalateur selon la revendication 2, caractérisé en ce que les communications respectives s'ouvrent un peu avant la fin de chacune des phases successives.

4. Inhalateur selon la revendication 3, caractérisé en ce que ladite chambre de succion est en outre mise en communication avec l'extérieur par un passage convenablement étranglé, équilibrant en partie sa pression interne.

5. Inhalateur selon la revendication 4, caractérisé en ce que le passage étranglé (9a) communique avec l'extérieur par l'embouchure.

6. Inhalateur selon la revendication 4, caractérisé en ce que ledit passage est muni d'un clapet de refoulement.

7. Inhalateur selon la revendication 6, caractérisé en ce que ce clapet est constitué par une lèvre d'étanchéité (2a) du piston.

8. Inhalateur selon la revendication 2, caractérisé en ce que la mise en relation de la réserve avec la chambre de succion au passage des orifices est autorisée par un clapet, tiroir ou manchon (16, 17) que commande avec jeu le mouvement du piston.

9. Inhalateur selon la revendication 8, caractérisé en ce que ledit clapet (16, 18) joue un rôle de distributeur entre la chambre de succion (9-10) d'une part, la réserve (8) à travers la cavité (3) ou l'extérieur d'autre part.

## Claims

1. Powder product inhaler which essentially comprises a body (1) having an oral aspiration mouthpiece associated with a member (2) subject to alternate displacement serving as the distributing slide valve moved by a piston moving under two opposing actions, one being the repulsion action of an elastic means (6) and the other the attracting action, sad member carrying along its side wall at least one transfer cavity (3) which its movement will directly link at the end of repulsion to the lower outlet of a product reserve (8) fitted to the body, characterized in that at the end of attraction the powder transfer cavity is directly linked with the internal outlet of an external air intake (12) which the cavity consequently links by an adequate channel (13) to the mouthpiece, the attraction movement being due to the pressure drop caused by an aspiration on the part of the patient through the mouthpiece.

2. Inhaler according to claim 1, characterized in that the piston also closes a chamber (9-10), which its repulsion vents and that at the end of the repulsion travel, the cavity also links the reserve with said vacuumizing chamber.

3. Inhaler according to claim 2, characterized in that the respective communications open slightly before the end of each of the successive phases.

4. Inhaler according to claim 3, characterized in that said suction chamber is also linked with the exterior by an appropriately constricted passage partly balancing its internal pressure.

5. Inhaler according to claim 4, characterized in that the constricted passage (a) is linked with the exterior by the mouthpiece.

6. Inhaler according to claim 4, characterized in that said passage is provided with a delivery valve.

7. Inhaler according to claim 6, characterized in that said valve is constituted by a sealing lip (2a) of the piston.

8. Inhaler according to claim 2, characterized in that the linking of the reserve with the suction chamber on passing the orifices is authorized by a valve, slide valve or sleeve (16, 17) controlled with clearance by the movement of the piston.

9. Inhaler according to claim 8, characterized in that said valve (16, 18) serves as a distributor between the suction chamber (9-10) on the one hand and the reserve (8) through the cavity (3) or the exterior on the other.

## Patentansprüche

1. Inhalator für pulverförmige Produkte, welcher im wesentlichen aus einem Gehäuse (1) besteht, das mit einer Mundansaugmündung ausgerüstet ist, verbunden mit einem hin- und hergehenden Bewegungselement (2), das die Aufgabe eines von einem Kolben angetriebenen Schiebers hat und unter zwei einander entgegengesetzten Wirkungen beweglich ist, davon ist eine durch ein elastisches Mittel (6) rückstoßend und die andere anziehend, wobei dieses Element entlang seiner Seitenwand wenigstens einen Transporthohlraum (3) trägt, durch welchen seine Bewegung am Ende des Rückstoßes in direkte Beziehung zum unteren Ausgang eines auf dem Gehäuse befestigten Produktvorrats (8) gebracht wird, **dadurch gekennzeichnet, daß** am Ende des Anziehvorgangs der Transporthohlraum für das Pulver in direkte Beziehung zum unteren Ausgang einer Lüftungsöffnung (12) für Umgebungsluft gebracht und daß der Hohlraum so über einen entsprechenden Kanal (13) zu der Mündung in Beziehung gebracht wird, wobei die Anziehbewegung auf den Unterdruck zurückzuführen ist, welcher von der Einatmung des Patienten durch die Mündung erzeugt wird.

2. Inhalator nach Anspruch 1, **dadurch gekennzeichnet, daß** der Kolben ebenfalls eine Kammer (9, 10) verschließt, in welcher durch seinen Rückstoß ein Vakuum erzeugt wird, und daß durch den Hohlraum am Ende des Rückstoßweges auch der Vorrat mit dieser unter Vakuum stehenden Kammer in Beziehung gebracht wird.

3. Inhalator nach Anspruch 2, **dadurch gekennzeichnet, daß** sich die jeweiligen Verbindungen bereits etwas vor dem Ende jeder der aufeinanderfolgenden Phasen öffnen.

4. Inhalator nach Anspruch 3, **dadurch gekennzeichnet, daß** die Ansaugkammer außerdem mittels eines geeignet verengten Durchlasses mit der Umgebung in Verbindung gebracht wird, wodurch sich teilweise ihr Innendruck ausgleicht.

5. Inhalator nach Anspruch 4, **dadurch gekennzeichnet, daß** der enge Durchlaß (9a) über die Mündung mit der Umgebung in Verbindung steht.

6. Inhalator nach Anspruch 4, **dadurch gekennzeichnet, daß** der Durchlaß mit einem Rückschlagventil ausgerüstet ist.

7. Inhalator nach Anspruch 6, **dadurch gekennzeichnet, daß** das Rückschlagventil aus einer Dichtungsfase (2a) des Kolbens gebildet ist.

8. Inhalator nach Anspruch 2, **dadurch gekennzeichnet, daß** das In-Beziehung-Bringen des Vorrats mit der Ansaugkammer über den Durchgang der Öffnungen durch ein Rückschlagventil, einen Schieber oder eine Hülse (16, 17), welche die Kolbenbewegung mit Spiel steuern, ermöglicht wird.

9. Inhalator nach Anspruch 8, **dadurch gekennzeichnet, daß** das Rückschlagventil (16, 18) die Aufgabe eines Verteilers zwischen der Ansaugkammer (9, 10) auf der einen und dem Vorrat (8) durch den Hohlraum (3) oder die Umgebung auf der anderen Seite übernimmt.
